Europäisches Patentamt

European Patent Office

Office européen des brevets

(19)

(11) Veröffentlichungsnummer : **0 179 296**
**B1**

(12) **EUROPÄISCHE PATENTSCHRIFT**

(45) Veröffentlichungstag der Patentschrift :
26.04.89

(51) Int. Cl.⁴ : **G 01 N 33/28**, G 01 N 1/22

(21) Anmeldenummer : **85112152.5**

(22) Anmeldetag : **25.09.85**

(54) Vorrichtung zur Ermittlung der quantitativen Zusammensetzung von Gasen.

(30) Priorität : **25.10.84 CH 5100/84**

(43) Veröffentlichungstag der Anmeldung :
**30.04.86 Patentblatt 86/18**

(45) Bekanntmachung des Hinweises auf die Patenterteilung : **26.04.89 Patentblatt 89/17**

(84) Benannte Vertragsstaaten :
**AT CH DE FR GB IT LI NL SE**

(56) Entgegenhaltungen :
**EP—A— 0 017 106**
**FR—A— 2 363 111**
**US—A— 4 409 814**

(73) Patentinhaber : **BBC Brown Boveri AG**
**Haselstrasse**
**CH-5401 Baden (CH)**

(72) Erfinder : **Knab, Hans-Josef, Dr. Phys.**
**Kilchbergstrasse 127**
**CH-8038 Zürich (CH)**

## Beschreibung

Die vorliegende Erfindung betrifft eine Vorrichtung zur Ermittlung der quantitativen Zusammensetzung von Gasen gemäss dem Oberbegriff des Anspruchs 1.

Aus der Druckschrift « Betriebsüberwachung durch Untersuchungen des Isolieröls » von Dörnenburg und Hutzel (etz-a Bd. 98, 1977, Seiten 211 bis 215) ist bereits eine derartige Vorrichtung zur Ermittlung der Zusammensetzung von Gasen bekannt, welche die Methode der Gasextraktion aus Isolierölproben unter Unterdruck benutzt, um die für eine Analyse nötigen Gasmengen zu erhalten.

Vor dem Abziehen der eigentlichen Isolierölprobe wird hierbei soviel Vorspülöl entnommen, dass sichergestellt ist, dass die Isolierölprobe aus dem Kesselinneren des elektrischen Gerätes stammt, und nicht aus Zuleitungen und Hähnen. Um Gasverluste und Verfälschungen durch probenfremde Aussenluft zu vermeiden, wird die Isolierölprobe zweckmässigerweise unter Luftabschluss in einen evakuierten Probenbehälter abgezogen. Dieser Probenbehälter wird nun, häufig über grosse Entfernungen, in ein stationäres Laboratorium transportiert.

Hier wird das Isolieröl aus dem Probenbehälter in ein grösseres, evakuiertes Gefäss gefüllt, dabei werden im Isolieröl gelöste Gase frei. Diese freiwerdenden Gase werden fortwährend mittels einer Toepler-Pumpe in ein Gassammelgefäss gepumpt, solange bis das Isolieröl vollständig entgast ist. Diese Gase werden anschliessend in einem Gaschromatographen analysiert.

Es erweist sich hierbei als nachteilig, dass zwischen Entnahmeort der Isolierölprobe und dem stationären Laboratorium grosse Entfernungen liegen, so dass mit erheblichen Wartezeiten bis zum Vorliegen der Analysenresultate gerechnet werden muss.

Ferner ist aus dem Firmenprospekt der Firma Pennsylvania Transformer Division ein tragbarer Gasdetektor bekannt, welcher erlaubt den Anteil brennbarer Gase im Gaskissen eines isolierölgefüllten elektrischen Gerätes zu bestimmen. Mit diesem Gasdetektor ist es jedoch nicht möglich, den quantitativen Anteil jeder Gaskomponente zu bestimmen. Zudem ist er nur bei Geräten einsetzbar, welche über ein Gaskissen verfügen.

Es ist insbesondere Aufgabe der Erfindung eine transportable Vorrichtung zu schaffen, welche es in einfacher und sicherer Weise erlaubt, die quantitative Zusammensetzung der im Isolieröl von elektrischen Geräten gelösten Gase, schnell und zuverlässig zu bestimmen.

Die Erfindung, wie sie in den Ansprüchen gekennzeichnet ist, löst diese Aufgabe.

Durch die erfindungsgemässe Vorrichtung wird erreicht, dass innerhalb kürzester Frist vor Ort die quantitative Zusammensetzung der im Isolieröl gelösten Gase bestimmt werden kann. Basierend auf diese Analysenresultate kann der Zustand der Oelisolation des kontrollierten elektrischen Gerätes hinreichend genau beurteilt werden, um sofort Aussagen über seine Betriebssicherheit machen zu können.

Ferner ist es vorteilhaft, dass der Isolierölprobentransport über weite Strecken in der Regel nicht mehr nötig ist, so dass die Gefahr einer Verfälschung der Isolierölprobe mit probenfremder Aussenluft entfällt.

Für gültige Messresultate wird relativ wenig Isolieröl benötigt, so dass auch Messwandler, die geringe Mengen Isolieröl enthalten, mit der erfindungsgemässen Vorrichtung untersucht werden können, ohne die Betriebssicherheit des Messwandlers durch die Isolierölentnahme zu gefährden.

Bei der Oelprobenentnahme wird stets die gleiche Menge Isolieröl entnommen und während der gleichen Zeitdauer entgast. Auf diese Weise erhält man sehr gute Vergleichsunterlagen für isolierölgefüllte elektrische Geräte, die während ihres Lebensalters routinemässig kontrolliert werden, so dass Unregelmässigkeiten im Gashaushalt sofort auffallen müssen.

In der Zeichnung sind Ausführungsbeispiele der Erfindung schematisch dargestellt.

Es zeigt :

Fig. 1 eine erste beispielsweise Ausführungsform der Erfindung, und

Fig. 2 eine zweite beispielsweise Ausführungsform der Erfindung.

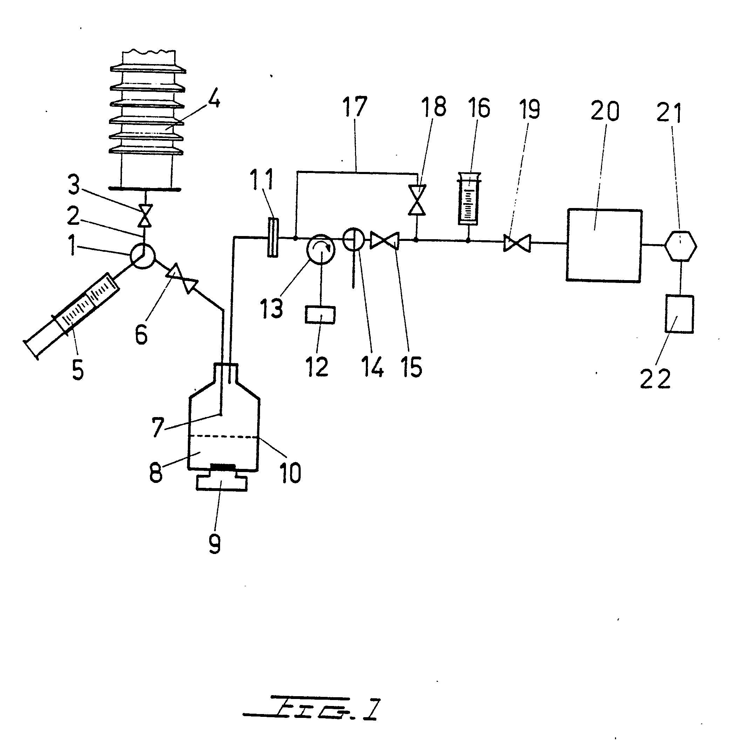

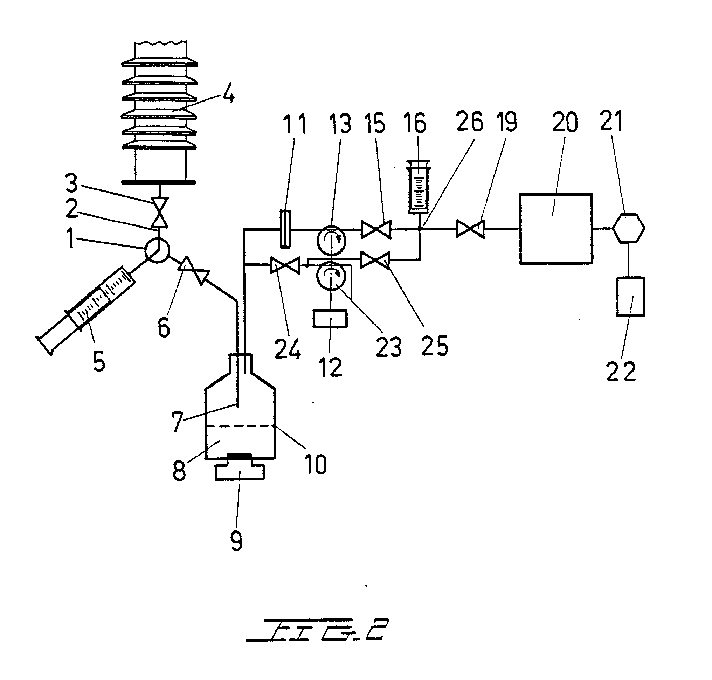

Gleiche Teile sind in Fig. 2 mit denselben Bezugszeichen wie in Fig. 1 versehen.

Die erfindungsgemässe Vorrichtung entsprechend Fig. 1 weist ein Umschaltventil 1 auf, dessen Eingang druckdicht über ein flexibles Leitungsstück 2 mit einem Ablasshahn 3 eines ölisolierten elektrischen Gerätes 4, wie etwa einem Stromwandler, verbunden ist. An dem einen Ausgang des Umschaltventils 1 ist eine Vorspülspritze 5 befestigt, der andere Ausgang führt über ein Absperrventil 6 durch eine Düse 7 in ein durchsichtiges Extraktionsgefäss 8. In den Boden des Extraktionsgefässes 8 ist ein Rührer 9, vorzugsweise ein Magnetrührer, eingelassen und an dessen Seitenwand ist eine Füllstandsmarkierung 10 angebracht. Aus dem Extraktionsgefäss 8 führt eine Leitung über einen Oelabscheidefilter 11 zum Eingang einer von einem Antriebsmodul 12 angetriebenen Schlauchpumpe 13. Der Ausgang der Schlauchpumpe 13 ist mit einem Umschaltventil 14 verbunden, welches den Ausgang der Schlauchpumpe 13 wahlweise mit Aussenluft oder über ein Absperrventil 15, mit dem Eingang eines Gassammelgefässes 16 verbindet. Zwischen dem Eingang des Gassammelgefässes 16 und dem Eingang der Schlauchpumpe 13 ist eine Bypassleitung 17 geschaltet, die mittels eines Absperrventils 18 geöffnet oder unterbrochen werden kann. Vom Eingang des Gassammelgefässes 16 geht eine weitere Leitung über ein Absperrventil 19 in einen transportablen Gaschromatographen 20, der in Verbindung steht mit einem Flammenionisationsdetektor 21 und einem Schreiber 22.

Zur Erläuterung der Wirkungsweise sei Fig. 1 näher betrachtet. Bei geschlossenem Absperrventil 6 und offenem Ablasshahn 3 wird aus dem elektrischen Gerät 4 mit der Vorspülspritze 5 eine hinreichende Menge Vorspülöl abgezogen. Der Bereich zwischen den geschlossenen Absperrventilen 6 und 19 wird von der Schlauchpumpe 13 evakuiert, dabei ist der Ausgang der Schlauchpumpe 13 über das Umschaltventil 14 an Aussenluft gelegt und das Absperrventil 15 ist geschlossen, das Absperrventil 18 ist offen.

Nach beispielsweise dreissig Minuten Pumpzeit ist ein Enddruck von 100 bis 500 Pa erreicht. Das Umschaltventil 14 wird umgeschaltet, das Absperrventil 15 wird geöffnet und das Absperrventil 18 geschlossen. Nach dem Umschalten des Umschaltventiles 1 und dem Oeffnen des Absperrventiles 6 strömt Isolieröl aus dem Kesselinneren des elektrischen Gerätes 4 zum Extraktionsgefäss 8. Beim Eintritt in das Extraktionsgefäss 8 wird das Isolieröl durch die Düse 7 versprüht. Die im Isolieröl gelösten Gase werden extrahiert und von der Schlauchpumpe 13 durch den Oelabscheidefilter 11, der aufgewirbelten Oelpartikeln den Zutritt zur Schlauchpumpe 13 verwehrt, in das Gassammelgefäss 16 gepumpt. Beim Erreichen der Füllstandsmarkierung 10, wird die weitere Isolierölzufuhr durch das Schliessen des Absperrventils 6 unterbunden. Während des Extrahierens wird das Isolieröl im Extraktionsgefäss durch einen Rührer 9 umgewälzt. Der Pumpvorgang wird nach beispielsweise zehn Minuten abgebrochen, wenn sich der überwiegende Teil der extrahierten Gase im Gassammelgefäss 16 befindet, und das Absperrventil 15 wird geschlossen. Das Vorspülöl wird mittels der Vorspülspritze 5 durch das entsprechend geschaltete Umschaltventil 1 und den Ablasshahn 3 in das elektrische Gerät 4 zurückgedrückt.

Nach dem Oeffnen des Absperrventils 19 gelangt das Gasgemisch aus dem Gassammelgefäss 16 in den Gaschromatographen 20 und wird mit Hilfe des Flammenionisationsdetektors 21 analysiert. Die Analysenresultate registriert der Schreiber 22.

Die erfindungsgemässe Vorrichtung gemäss Fig. 2 unterscheidet sich von derjenigen nach Fig. 1 dadurch, dass zwei eingangsseitig miteinander verbundene Leitungsstränge zu je einer Schlauchpumpe 13, 23 führen. Der ersten Schlauchpumpe 13 ist ein Oelabscheidefilter 11 vorgeschaltet, während deren Ausgang über ein Absperrventil 15 zu einem Leitungsknoten 26 führt. Dem Eingang der zweiten Schlauchpumpe 23 ist ein Absperrventil 24 vorgeschaltet, während ihr Ausgang mit der Aussenluft in Verbindung steht. Der Eingang der zweiten Schlauchpumpe 23 ist über eine unterbrechbare Verbindungsleitung mit dem Leitungsknoten 26 verbunden. Mit dem Leitungsknoten 26 ist ferner ein Gassammelgefäss 16 und ein Zugang zu einem Absperrhahn 19 verbunden. Die beiden Schlauchpumpen 13, 23 werden von einem gemeinsamen Antriebsmodul 12 über eine gemeinsame Welle angetrieben.

Die Wirkungsweise der Vorrichtung gemäss Fig. 2 ist ähnlich wie die der Vorrichtung gemäss Fig. 1. Bei der Evakuierung des Bereiches zwischen den geschlossenen Absperrventilen 6 und 19 sind die Absperrventile 15, 24 und 25 offen. Nach beispielsweise zehn Minuten Pumpzeit ist ein Enddruck von 100 bis 500 Pa erreicht, und die Absperrventile 24 und 25 werden geschlossen. Diese gegenüber der Anordnung gemäss Fig. 1 vorteilhaft kurze Pumpzeit wird dadurch erreicht, dass beim Evakuierungsvorgang der Oelabscheidefilter 11 teilweise überbrückt ist. Nach dem Oeffnen des Absperrventils 6 strömt, wie bereits beschrieben, Isolieröl in das Extraktionsgefäss 8. Die Schlauchpumpe 13 befördert das extrahierte Gas in das Gassammelgefäss 16. Nach Beendigung des Extrahierens wird das Absperrventil 15 geschlossen. Nach dem Oeffnen des Absperrventils 19 erfolgt, wie bereits beschrieben, die Analyse des im Gassammelgefäss 16 vorhandenen Gasgemisches.

Soll die Vorrichtung für einen niedrigeren Enddruck bemessen sein, so können statt der jeweils einen Schlauchpumpe auch jeweils zwei oder mehrere Schlauchpumpen in Serie eingebaut werden.

Bei ölisolierten elektrischen Geräten, welche über der Oelfüllung ein Gaskissen aufweisen, kann das Gasgemisch unter Luftabschluss direkt entnommen und in das Gassammelgefäss gepumpt werden, von dort gelangt es, wie bereits geschildert, in den Gaschromatographen zur Analyse. Ferner kann diese Vorrichtung auch zur Entnahme von Isolierölproben verwendet werden, wenn statt des Extraktionsgefässes ein entsprechender Probenbehälter eingesetzt wird.

Die erfindungsgemässe Vorrichtung ist betriebsbereit und vor Transportschäden geschützt, in einem oder mehreren nicht dargestellten, kofferartigen Gehäusen untergebracht. In den Gehäusen sind über den Bedienungsöffnungen mit Schnellverschlüssen versehene Abdeckungen vorgesehen. Die für den Betrieb der Vorrichtung nötige elektrische Energie wird nicht dargestellten Batterien entnommen, welche innerhalb der Gehäuse auswechselbar montiert sind.

Die Vorrichtung kann fernsteuerbar ausgestaltet werden. Dies kann dadurch erreicht werden, dass die Vorspülspritze durch eine fernsteuerbare elektrische Pumpe ersetzt wird, dass eine weitere zusätzliche Pumpe die Gase aus dem Gassammelgefäss in den Gaschromatographen pumpt, und dass die Schlauchpumpen ferngesteuert betätigbar ausgebildet sind. Ferner kann jedes der Absperrventile mit einer ferngesteuerten elektromagnetischen Betätigung ausgerüstet werden. Damit ist es möglich den Zustand des Isolieröls zu überwachen, ohne das betreffende elektrische Gerät ausser Betrieb nehmen zu müssen, und ohne dass sich das Messpersonal während der Messung bei der Vorrichtung, unmittelbar neben dem betroffenen elektrischen Gerät, aufhalten muss.

**Patentansprüche**

1. Vorrichtung zur Ermittlung der quantitativen Zusammensetzung von Gasen, welche im Isolieröl von ölisolierten elektrischen Geräten gelöst sind, mit einer Entnahmeeinrichtung für Isolierölproben, einem Gasextraktionsteil zur Extraktion der Gase aus dem Isolieröl mittels Unterdruck und zum Transport der extrahierten Gase in ein Gassammelgefäss, und einem Gaschromatographen zur Analyse der im Gassammelgefäss befindlichen Gase, dadurch gekennzeichnet, dass die Vorrichtung als transportable und autark arbeitende Baugruppe ausgebildet ist und mindestens eine peristaltisch wirkende Pumpenanordnung aufweist, deren Eingang über das Gasextraktionsteil mit der Entnahmeeinrichtung und deren Ausgang wahlweise mit der Aussenluft oder mit dem Gassammelgefäss verbindbar ist.

2. Vorrichtung nach Anspruch 1, dadurch gekennzeichnet, dass die peristaltisch wirkende Pumpenanordnung mindestens eine Schlauchpumpe (13) enthält, deren Ausgang wahlweise mit Aussenluft oder mit dem Gassammelgefäss (16) verbindbar ist, und dass zwischen deren Eingang und dem Eingang des Gassammelgefässes (16) eine unterbrechbare Bypassleitung (17) geschaltet ist.

3. Vorrichtung nach Anspruch 2, dadurch gekennzeichnet, dass dem Eingang der Schlauchpumpe (13) ein Oelabscheidefilter (11) vorgeschaltet ist.

4. Vorrichtung nach einem der Ansprüche 2 oder 3, dadurch gekennzeichnet, dass zwischen Ausgang der Schlauchpumpe (13) und dem Gassammelgefäss (16) ein Umschaltventil (14) und ein diesem nachgeschaltetes Absperrventil (15) angeordnet sind.

5. Vorrichtung nach Anspruch 1, dadurch gekennzeichnet, dass die peristaltisch wirkende Pumpenanordnung mindestens zwei Schlauchpumpen (13, 23) enthält, die eingangsseitig miteinander verbunden sind, dass der Ausgang einer ersten (13) beider Schlauchpumpen (13, 23) mit dem Eingang einer zweiten (23) beider Schlauchpumpen (13, 23) und dem Gassammelgefäss (16) verbindbar ist, und dass der Ausgang der zweiten Schlauchpumpe (23) mit der Aussenluft in Verbindung steht.

6. Vorrichtung nach Anspruch 5, dadurch gekennzeichnet, dass die mindestens zwei Schlauchpumpen (13, 23) auf einer gemeinsamen Antriebswelle sitzen.

7. Vorrichtung nach Anspruch 5, dadurch gekennzeichnet, dass der ersten Schlauchpumpe (13) ein Oelabscheidefilter (11) vorgeschaltet ist.

8. Vorrichtung nach einem der Ansprüche 5 bis 7, dadurch gekennzeichnet, dass zwischen dem Ausgang der ersten Schlauchpumpe (13) und Gassammelgefäss (16), sowie zwischen Gassammelgefäss (16) und Eingang der zweiten Schlauchpumpe (23) und zwischen Extraktionsgefäss (8) und Eingang der zweiten Schlauchpumpe (23) jeweils mindestens ein Absperrventil (z. B. 25) vorgesehen ist.

9. Vorrichtung nach einem der Ansprüche 1 bis 8, dadurch gekennzeichnet, dass zu der mindestens einen (13) oder zu mindestens einer der mindestens zwei Schlauchpumpen (13, 23) eine weitere Schlauchpumpe in Serie geschaltet ist.

10. Vorrichtung nach einem der Ansprüche 2 bis 9, dadurch gekennzeichnet, dass die mindestens eine (13) oder die mindestens zwei Schlauchpumpen (13, 23) fernsteuerbar ausgebildet sind.

11. Vorrichtung nach einem der Ansprüche 2 bis 10, dadurch gekennzeichnet, dass die mindestens eine Schlauchpumpe (13) bzw. die mindestens zwei Schlauchpumpen (13, 23) in einem kofferartigen Gehäuse eingebaut ist bzw. sind.

**Claims**

1. Device for the determination of the quantitative composition of gases which are dissolved in the insulating oil of oil-insulated electrical apparatus, with a device for removing insulating oil samples, a gas extraction section for the extraction of the gases from the insulating oil by means of reduced pressure and for conveying the extracted gases to a gas collecting vessel, and a gas chromatograph for the analysis of the gases in the gas collecting vessel, characterised in that the device is designed as a transportable and autarkically operating assembly and incorporates at least one peristaltically operating pump arrangement whose inlet can be connected via the gas extraction section to the removal device and whose outlet can be connected optionally to the outside air or to the gas collecting vessel.

2. Device according to Claim 1, characterised in that the peristaltically operating pump arrangement contains at least one peristaltic pump (13) whose outlet can be connected optionally with the outside air or with the gas collecting vessel (16), and that between the inlet of which and the inlet of the gas collecting vessel (16) there is connected a bypass pipe (17) which can be shut off.

3. Device according to Claim 2, characterised in that the inlet of the peristaltic pump (13) has an oil separating filter (11) inserted in front of it.

4. Device according to one of the Claims 2 or 3, characterised in that a changeover valve (14) and a stopcock (15) inserted after the latter are disposed between the outlet of the peristaltic pump (13) and the gas collecting vessel (16).

5. Device according to Claim 1, characterised in that the peristaltically operating pump arrangement comprises at least two peristaltic pumps (13, 23) which are connected together on the inlet side, that the outlet of a first (13) of two peristaltic pumps (13, 23) can be connected to the inlet of a second (23) of two peristaltic pumps (13, 23) and to the gas collecting vessel (16), and that the outlet of the second peristaltic pump (23) is connected to the outside air.

.6. Device according to Claim 5, characterised in that the peristaltic pumps (13, 23) at least two in number are mounted on a common drive shaft.

7. Device according to Claim 5, characterised in that the first peristaltic pump (13) has an oil separating filter (11) inserted in front of it.

8. Device according to one of the Claims 5 to 7, characterised in that in each case at least one stopcock (e. g. 25) is provided between the outlet of the first peristaltic pump (13) and the gas collecting vessel (16), and between the gas collecting vessel (16) and the inlet of the second peristaltic pump (23), and between the extraction vessel (8) and the inlet of the second peristaltic pump (23).

9. Device according to one of the Claims 1 to 8, characterised in that a further peristaltic pump is connected in series with the peristaltic pump (13) at least one in number or with at least one of the peristaltic pumps (13, 23) at least two in number.

10. Device according to one of the Claims 2 to 9, characterised in that the at least one (13) or the at least two peristaltic pumps (13, 23) are constructed for remote control.

11. Device according to one of the Claims 2 to 10, characterised in that the at least one peristaltic pump (13) or the at least two peristaltic pumps (13, 23) is or are installed in a case-like housing.

**Revendications**

1. Dispositif pour déterminer la composition quantitative de gaz qui sont dissous dans l'huile isolante d'appareils électriques isolés par de l'huile, comportant un dispositif de prélèvement d'échantillons d'huile isolante, un moyen d'extraction de gaz pour extraire les gaz de l'huile isolante à l'aide d'une dépression et pour transporter les gaz extraits dans un récipient collecteur de gaz, et un chromatographe en phase gazeuse pour analyser les gaz présents dans le récipient collecteur de gaz, caractérisé en ce qu'il a la forme d'un ensemble transportable et fonctionnant de manière autonome et comporte au moins un dispositif de pompage à fonctionnement péristaltique, dont l'entrée peut être raccordée, par l'intermédiaire du moyen d'extraction de gaz, au dispositif de prélèvement et dont la sortie peut être raccordée sélectivement à l'atmosphère ou au récipient collecteur de gaz.

2. Dispositif suivant la revendication 1, caractérisé en ce que le dispositif de pompe à fonctionnement péristaltique comprend au moins une pompe péristaltique (13) dont la sortie peut être raccordée sélectivement à l'atmosphère ou au récipient collecteur de gaz (16), et qu'entre son entrée et l'entrée du récipient collecteur de gaz (16) est branchée une dérivation (17) obturable.

3. Dispositif suivant la revendication 2, caractérisé en ce qu'un filtre séparateur d'huile (11) précède l'entrée de la pompe péristaltique (13).

4. Dispositif suivant la revendication 2 ou 3, caractérisé en ce qu'entre la sortie de la pompe péristaltique (13) et le récipient collecteur de gaz (16) sont installées une valve de commutation (14) et une valve d'arrêt (15) montée en aval de celle-ci.

5. Dispositif suivant la revendication 1, caractérisé en ce que le dispositif de pompage à fonctionnement péristaltique comprend au moins deux pompes péristaltiques (13, 23) qui sont raccordées l'une à l'autre par leurs entrées, que la sortie d'une première (13) des deux pompes péristaltiques (13, 23) peut être raccordée à l'entrée d'une seconde (23) des deux pompes péristaltiques (13, 23) et au récipient collecteur de gaz (16), et que la sortie de la seconde pompe péristaltique (23) est en communication avec l'atmosphère.

6. Dispositif suivant la revendication 5, caractérisé en ce que les pompes péristaltiques qui sont au moins au nombre de deux (13, 23) sont montées sur un arbre d'entraînement commun.

7. Dispositif suivant la revendication 5, caractérisé en ce que la première pompe péristaltique (13) est précédée d'un filtre séparateur d'huile (11).

8. Dispositif suivant l'une quelconque des revendications 5 à 7, caractérisé en ce qu'entre la sortie de la première pompe péristaltique (13) et le récipient collecteur de gaz (16) ainsi qu'entre le récipient collecteur de gaz (16) et l'entrée de la seconde pompe péristaltique (23) et entre le récipient d'extraction (8) et l'entrée de la seconde pompe péristaltique (23) est chaque fois prévue au moins une valve d'arrêt (par exemple 25).

9. Dispositif suivant l'une quelconque des revendications 1 à 8, caractérisé en ce qu'une autre pompe péristaltique est raccordée en série à la pompe péristaltique au moins unique (13) ou à au moins une des pompes péristaltiques qui sont au moins au nombre de deux (13, 23).

10. Dispositif suivant l'une quelconque des revendications 2 à 9, caractérisé en ce que la pompe péristaltique au moins unique (13) ou les pompes péristaltiques qui sont au moins au nombre de deux (13, 23) sont propres à être télécommandées.

11. Dispositif suivant l'une quelconque des revendications 2 à 10, caractérisé en ce que la pompe péristaltique au moins unique (13) ou les pompes péristaltiques qui sont au moins au nombre de deux (13, 23) est ou sont installées dans un boîtier en forme de coffret.

_FIG. 1_

FIG. 2